# EUROPÄISCHE PATENTSCHRIFT

(11) **EP 2 283 768 B1**
(45) Veröffentlichungstag und Bekanntmachung des Hinweises auf die Patenterteilung: **05.10.2016**
(21) Anmeldenummer: 10008133.0
(22) Anmeldetag: 04.08.2010
(51) Int. Cl.: A61B 1/00, A61B 1/07, G02B 23/24

(54) **Vorrichtung und Verfahren zur Zentrierung von Innen- und Aussenrohr eines Endoskops**
Device and method for centring the internal and external tube of an endoscope
Dispositif et procédé de centrage d'un tube extérieur et intérieur d'un endoscope

(30) Priorität: 14.08.2009 DE 102009037317
(43) Veröffentlichungstag der Anmeldung: 16.02.2011
(73) Patentinhaber: Karl Storz GmbH & Co. KG, 78532 Tuttlingen (DE)
(72) Erfinder: Dahmen, Jan, 78532 Tuttlingen (DE)

(56) Entgegenhaltungen:
- EP-A2- 0 416 371
- DE-A1- 10 307 903
- JP-A- 2001 017 381
- US-A- 4 569 335
- US-A- 4 981 390
- US-A- 5 651 759
- US-A1- 2005 119 525

## Beschreibung

Die vorliegende Erfindung bezieht sich auf eine Positionierungseinrichtung zur Positionierung eines Innenrohrs in einem Außenrohr eines Endoskops, ein Endoskop mit einer solchen Positionierungseinrichtung und ein Verfahren zum Positionieren eines Innenrohrs in einem Außenrohr eines Endoskops.

Der Schaft eines Endoskops weist oft mehrere ineinander angeordnete Rohre auf. In den Hohlräumen zwischen diesen Rohren sind beispielsweise Lichtwellenleiter zur Übertragung von Licht zur Beleuchtung eines zu untersuchenden Hohlraums angeordnet. Bei einem medizinischen Eingriff kann der Schaft eines starren Endoskops durch das medizinische Personal einer erheblichen mechanischen Belastung ausgesetzt werden, insbesondere einer Biegebelastung. Erhebliche mechanische Spannungen können auch während des Autoklavierens aufgrund unterschiedlicher thermischer Ausdehnungskoeffizienten verschiedener Bauteil oder aufgrund von Temperaturgradienten entstehen.

Ein mechanisches Verbiegen eines Rohrs aufgrund entsprechender mechanischer Belastung oder auch aufgrund thermischer Spannungen während des Autoklavierens verformt die Hohlräume zwischen den Rohren. In den Hohlräumen angeordnete Lichtwellenleiter, elektrische Leiter oder andere Funktionselemente können dadurch verschoben, gedehnt, gestaucht, gequetscht, zerrieben oder auf andere Weise beschädigt werden. Außerdem kann ein Verbiegen der Rohre und die resultierende Verschiebung von Lichtwellenleitern eine einseitige und ungleichmäßige Beleuchtung des Raums vor dem distalen Ende des Endoskops zur Folge haben

Um eine Verformung von Hohlräumen zwischen ineinander angeordneten Rohren eines Schafts eines Endoskops zu vermeiden, werden herkömmlich im Wesentlichen in Längsrichtung des Schafts verlaufende Drahtstücke oder Flügelsegmente an das jeweils innere Rohr gelötet, geschweißt oder geklebt. Zwischen den Drahtstücken oder Flügelsegmenten können Lichtwellenleiter und andere Einrichtungen im Zwischenraum zwischen den Rohren angeordnet werden. Die Drahtstücke oder Flügelsegmente dienen der mechanischen Abstützung des jeweils inneren Rohres gegenüber dem jeweils äußeren Rohr. Dadurch wird eine Verformung des Zwischenraums vermindert und durch die Verteilung mechanischer Spannungen auf beide Rohre die Steifheit des Schafts erhöht.

Eine ähnliche Anordnung ist in EP 0 416 371 A2 beschrieben. Ein Endoskop umfasst ein optische Bauelemente aufnehmendes Innenrohr und ein das Innenrohr umgebendes Außenrohr. Der Zwischenraum zwischen Innenrohr und Außenrohr wird durch in Längsrichtung verlaufende Stege unterteilt, die das Innenrohr im Außenrohr zentrieren.

Das Anordnen und Fügen der Drahtstücke oder Flügelsegmente am jeweils inneren Rohr ist jedoch zeitaufwendig und arbeits- und damit kostenintensiv. Ferner müssen die an das innere Rohr gefügten Flügelsegmente anschließend an die Innenkontur des Außenrohrs angepasst werden, beispielsweise durch Überdrehen. Dabei können die Drahtstücke oder Flügelsegmente wieder abgerissen werden.

Im Dokument JP 2001-17381 A ist ein Schaft eines Endoskops beschrieben, der in einem Zwischenraum zwischen einem Außenrohr und einem Optikrohr ein weiteres Rohr, das als Innenrohr bezeichnet wird, aufweist. Das Innenrohr erstreckt sich über die gesamte Länge des Schafts.

Eine Aufgabe der vorliegenden Erfindung besteht darin, eine verbesserte Positionierungseinrichtung, ein verbessertes Endoskop und ein verbessertes Verfahren zum Positionieren eines Innenrohrs in einem Außenrohr eines Endoskops zu schaffen.

Diese Aufgabe wird durch die Gegenstände der unabhängigen Ansprüche gelöst.

Weiterbildungen sind in den abhängigen Ansprüchen angegeben.

Verschiedene Ausführungsformen der vorliegenden Erfindung beruhen auf der Idee, eine Positionierungseinrichtung zur zentrischen Positionierung eines Innenrohrs in einem Außenrohr eines Endoskops am Innenrohr oder am Außenrohr zu klemmen. An die Stelle der herkömmlichen stoffschlüssigen Verbindung tritt hier eine kraftschlüssige Verbindung. Dabei umgreift die Positionierungseinrichtung das Innenrohr zu mehr als der Hälfte. Die Klemmung erfolgt aufgrund der Elastizität der Positionierungseinrichtung. Die gesamte Positionierungseinrichtung oder aufgrund ihrer Form besonders elastische Abschnitte derselben bilden eine Klemmeinrichtung. Die Positionierungseinrichtung ist also für eine Klemmung am Innenrohr oder im Außenrohr ausgebildet.

Durch die Klemmung kann die Positionierungseinrichtung innerhalb kürzester Zeit montiert und danach sogar noch justiert werden. Die Geometrie der Positionierungseinrichtung ist bei ihrer Herstellung genau einstellbar. Anders als beim Schweißen, Löten oder Kleben bleibt bei der hier vorgesehenen bloßen Klemmung die Geometrie der Positionierungseinrichtung erhalten. Insbesondere ist die Lage der Außenkontur der Positionierungseinrichtung nach der Klemmung präzise definiert, da die Positionierungseinrichtung nicht teilweise aufgeschmolzen wird und auch kein Lot oder Klebstoff zwischen die Positionierungseinrichtung und das Innenrohr gelangen kann. Ein Überdrehen wie bei herkömmlich an das Innenrohr gefügten Drahtstücken oder Flügelsegmenten ist nicht erforderlich. Insgesamt resultiert ein deutlich verminderter Aufwand bei der Positionierung eines Innenrohrs in einem Außenrohr mittels der hier beschriebenen Positionierungseinrichtung. Ferner kann die Positionierungseinrichtung anders als beim Stand der Technik auch zunächst im Außenrohr geklemmt werden bevor das Innenrohr in das Außenrohr eingeführt wird.

Eine Positionierungseinrichtung zur Positionierung eines Innenrohrs in einem Außenrohr eines Schafts eines Endoskops umfasst zumindest eine Innenstützfläche zur Abstützung der Positionierungseinrichtung am Innenrohr, zumindest eine Außenstützfläche zur Abstützung der Positionierungseinrichtung am Außenrohr und eine Klemmeinrichtung zur Klemmung der Positionierungseinrichtung am Innenrohr oder am Außenrohr, wobei die Positionierungseinrichtung so ausgebildet ist, dass mehrere Positionierungseinrichtungen über die Länge des Schafts verteilt angeordnet werden können.

Die Positionierungseinrichtung ist ausgebildet. um das Innenrohr zu mehr als der Hälfte zu umgreifen. Die Positionierungseinrichtung kann mehrere voneinander beabstandete Innenstützflächen und/oder mehrere voneinander beabstandete Außenstützflächen aufweisen. Eine oder mehrere Innenstützflächen und eine bzw. mehrere Außenstützflächen können einander jeweils in radialer Richtung gegenüberliegen. Alternativ sind zumindest eine Innenstützfläche und zumindest eine Außenstützfläche in umfänglicher Richtung abwechselnd bzw. alternierend angeordnet, wobei Innenstützflächen und Außenstützflächen einander in umfänglicher Richtung nur wenig oder gar nicht überlappen. In diesem und anderen Fällen können einer oder mehrere Stege in radialer Richtung jeweils eine Innenstützfläche und eine Außenstützfläche verbinden. Eine Positionierungseinrichtung weist insbesondere mindestens drei derartige Stege als eigentliche Stützabschnitte zwischen Innenrohr und Außenrohr auf, wobei der Abstand zwischen benachbarten Stegen insbesondere weniger als 180° und vorteilhaft jeweils ca. 120° oder weniger beträgt.

Wenn die Positionierungseinrichtung nur eine Innenstützfläche oder nur eine Außenstützfläche aufweist, erstreckt sich diese in umfänglicher Richtung insbesondere über mehr als 180°. Wenn die Positionierungseinrichtung mehrere Innenstützflächen aufweist, ist der Abstand zwischen einander gegenüberliegenden Rändern von nächst benachbarten Innenstützflächen insbesondere kleiner als 180° oder kleiner als 120°, ggf. bezogen auf eine Symmetrieachse des Innenrohrs. Wenn die Positionierungseinrichtung mehrere Außenstützflächen aufweist, ist der Abstand zwischen einander gegenüberliegenden Rändern nächst benachbarter Außenstützflächen insbesondere kleiner als 180° oder kleiner als 120°, ggf. bezogen auf eine Symmetrieachse des Außenrohrs.

Ein Endoskop umfasst eine oder mehrere der oben beschriebenen Positionierungseinrichtungen. Auf das Innenrohr und die Positionierungseinrichtung kann ein Schrumpfschlauch aufgeschrumpft sein. Das Innenrohr und die Positionierungseinrichtung können umwickelt oder umsponnen sein, insbesondere mittels eines oder mehrerer Fäden, Drähte, Bänder oder faden- oder draht- oder bandartiger Einrichtungen.

Bei einem Verfahren zum Positionieren eines Innenrohrs in einem Außenrohr eines Schafts eines Endoskops wird zunächst eine Positionierungseinrichtung am Innenrohr oder im Außenrohr geklemmt, wobei die Positionierungseinrichtung eine Innenstützfläche zur Abstützung am Innenrohr und eine Außenstützfläche zur Abstützung am Außenrohr aufweist und so ausgebildet ist, dass mehrere Positionierungseinrichtungen über die Länge des Schafts verteilt angeordnet werden können. Nach dem Klemmen der Positionierungseinrichtung wird das Innenrohr im Außenrohr angeordnet. Bei einem weiteren Schritt vor oder nach dem Klemmen der Positionierungseinrichtung wird ein Lichtwellenleiter an der Außenwand des Innenrohrs oder an der Innenwand des Außenrohrs angeordnet. Die Positionierungseinrichtung kann ferner beim Klemmen oder nach dem Klemmen stoffschlüssig mit dem Innenrohr oder dem Außenrohr verbunden werden, beispielsweise durch Kleben oder Löten. Nach dem Klemmen der Positionierungseinrichtung am Innenrohr, insbesondere ggf. vor oder nach dem Anordnen eines Lichtwellenleiters an der Außenwand des Innenrohrs, kann ein Schrumpfschlauch auf das Innenrohr und die Positionierungseinrichtung aufgeschrumpft werden. Alternativ oder zusätzlich werden das Innenrohr und die Positionierungseinrichtung umwickelt oder umsponnen, beispielsweise mittels eines oder mehrerer Fäden, Drähte, Bänder oder fadenoder draht- oder bandartiger Einrichtungen.

Die vorliegende Erfindung ist insbesondere bei einem Innenrohr und einem Außenrohr mit jeweils kreisförmigem Querschnitt anwendbar, ist jedoch auch bei Rohren mit elliptischem, rechteckigem oder anderem Querschnitt anwendbar. Als Innenrohr wird hier das Rohr mit dem kleineren Querschnitt, als Außenrohr das Rohr mit dem größeren Querschnitt bezeichnet. Darüber hinaus kann das Endoskop ein oder mehrere Rohre innerhalb des Innenrohrs und/oder ein Rohr, in dem das Außenrohr angeordnet ist, aufweisen.

### Kurzbeschreibung der Figuren

Nachfolgend werden Ausführungsformen anhand der beigefügten Figuren näher erläutert. Es zeigen:
- Figur 1: eine schematische Darstellung eines Endoskops;
- Figur 2: eine schematische Darstellung eines Querschnitts und eines Längsschnitts eines Schafts eines Endoskops;
- Figur 3: eine schematische Darstellung eines Querschnitts und eines Längsschnitts eines Schafts eines Endoskops;
- Figur 4: eine schematische Darstellung von Querschnitten eines Schafts eines Endoskops und einer Positionierungseinrichtung;
- Figur 5: eine schematische Darstellung von Querschnitten eines Schafts eines Endoskops und einer Positionierungseinrichtung;
- Figur 6: eine schematische Darstellung von Querschnitten eines Schafts eines Endoskops und einer Positionierungseinrichtung;
- Figur 7: eine schematische Darstellung von Querschnitten eines Schafts eines Endoskops und einer Positionierungseinrichtung;
- Figur 8: ein schematisches Flussdiagramm.

### Beschreibung der Ausführungsformen

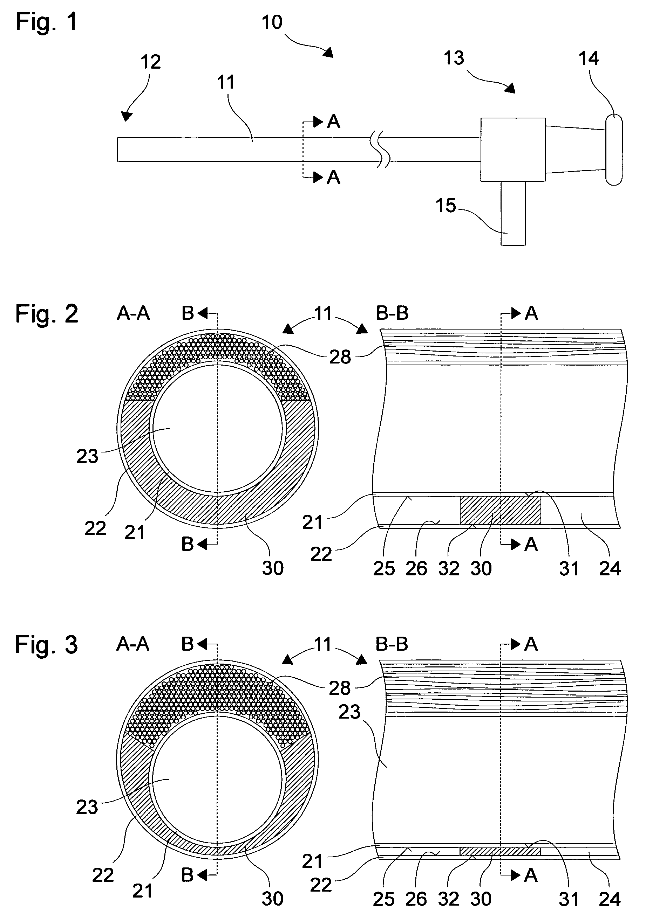

Figur 1 zeigt eine schematische Darstellung eines Endoskops 10 mit einem insbesondere zylindrischen oder kreiszylindrischen Schaft 11, einem distalen Ende 12 und einem proximalen Ende 13. Am proximalen Ende 13 weist das Endoskop 10 ein Okular 14 und eine Kupplung 15 zum Einkoppeln von Licht von einer Lichtquelle auf. Das Endoskop 10 kann weitere Einrichtungen aufweisen, die in Figur 1 nicht dargestellt sind.

Die nachfolgend beschriebenen Figuren 2 bis 7 zeigen jeweils Schnitte entlang der in Figur 1 gezeigten Ebene A-A durch verschiedene Ausführungsformen des Schafts 11. Die Ebene A-A liegt in allen Fällen senkrecht zur Achse oder zur Längsrichtung des Schafts 11. Die Figuren 2 und 3 zeigen jeweils schematische Darstellungen von Querschnitten und Längsschnitten durch den Schaft 11 einer Ausführungsform des oben anhand der Figur 1 dargestellten Endoskops 10. Dabei ist jeweils links ein Querschnitt entlang der Ebene A-A und rechts ein Längsschnitt entlang der Ebene B-B, die die Achse des Schafts 11 enthält, dargestellt. Der Schaft umfasst jeweils ein Innenrohr 21, das in einem Außenrohr 22 angeordnet ist. In einem Lumen 23 des Innenrohrs 21 ist beispielsweise eine Stablinsenoptik angeordnet, die in den Figuren nicht gezeigt ist. Der Schaft 11 kann ferner ein weiteres Rohr in dem Lumen 23 des Innenrohrs 21 und/oder ein weiteres Rohr, das das Außenrohr 22 umschließt, aufweisen.

In einem Zwischenraum 24 zwischen einer äußeren Oberfläche 25 des Innenrohrs 21 und einer inneren Oberfläche 26 des Außenrohrs 22 sind Lichtwellenleiter 28 und eine Positionierungseinrichtung 30 angeordnet. In den Querschnitten A-A ist erkennbar, dass die Positionierungseinrichtung 30 das Innenrohr jeweils zu mehr als der Hälfte umgreift. Eine Innenstützfläche 31 der Positionierungseinrichtung 30 liegt in einem zusammenhängenden Bereich an der äußeren Oberfläche 25 des Innenrohrs 21 an, der mehr als die Hälfte des äußeren Umfangs des Querschnitts des Innenrohrs 21 umfasst. Eine Außenstützfläche 32 der Positionierungseinrichtung 30 liegt in einem zusammenhängenden Bereich an der inneren Oberfläche 26 des Außenrohrs 22 an, der mehr als die Hälfte des inneren Umfangs des Querschnitts des Außenrohrs 22 umfasst. Der im dargestellten Querschnitt A-A nicht von der Positionierungseinrichtung 30 eingenommene Bereich des Zwischenraums zwischen dem Innenrohr 21 und dem Außenrohr 22 wird von in Längsrichtung des Schafts 11 verlaufenden Lichtwellenleitern 28 eingenommen.

Im Längsschnitt B-B ist jeweils erkennbar, dass die in Längsrichtung des Schafts 11 gemessene Länge der Positionierungseinrichtung 30 jeweils kleiner ist als der Außendurchmesser des Innenrohrs 21. Die Positionierungseinrichtung 30 kann jedoch von den Darstellungen in den Figuren 2 und 3 abweichend eine Länge aufweisen, die größer als der Außendurchmesser des Innenrohrs 21 ist. Insbesondere kann die Länge der Positionierungseinrichtung 30 ein Mehrfaches des Außendurchmessers des Innenrohrs 21 oder ein Mehrfaches des Innendurchmessers des Außenrohrs 22 betragen. Ferner kann die Positionierungseinrichtung 30 sich über mindestens ein Viertel, mindestens ein Drittel, mindestens die Hälfte oder auch über die gesamte Länge des Schafts 11 erstrecken.

Bei der in Figur 2 dargestellten Ausführungsform des Endoskops sind das Innenrohr 21 und das Außenrohr 22 koaxial bzw. konzentrisch zueinander angeordnet. Die Positionierungseinrichtung 30 hat damit insgesamt zumindest näherungsweise die Gestalt eines unvollständigen Kreisrings mit rechteckigem Querschnitt.

Bei der in Figur 3 dargestellten Ausführungsform des Schafts 11 des Endoskops 10 ist das Innenrohr 21 exzentrisch zum Außenrohr 22 angeordnet. Die Symmetrieachse des Innenrohrs 21 und des Außenrohrs 22 sind parallel zueinander und voneinander beabstandet. Der Abstand zwischen der äußeren Oberfläche 25 des Innenrohrs 21 und der inneren Oberfläche 26 des Außenrohrs 22 variiert deshalb in umfänglicher Richtung. Eine Folge ist, dass auch der Querschnitt (in einer Ebene parallel zu den Symmetrieachsen von Innenrohr 21 und Außenrohr 22) der Positionierungseinrichtung 30 in umfänglicher Richtung variiert. Eine andere Folge der exzentrischen Anordnung von Innenrohr 21 und Außenrohr 22 ist, dass der für die Lichtwellenleiter 28 verbleibende Raum größer ist. Es können deshalb im Vergleich zu der oben anhand der Figur 2 dargestellten Ausführungsform mehr Lichtwellenleiter 28 zwischen dem Innenrohr 21 und dem Außenrohr 22 angeordnet werden.

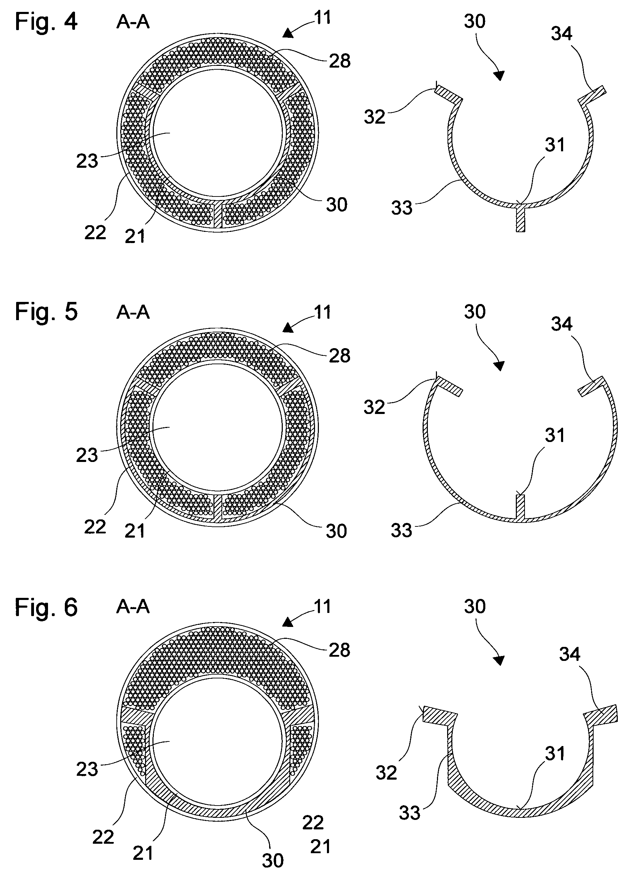

Die oben anhand der Figuren 2 und 3 dargestellten Positionierungseinrichtungen 30 liegen jeweils großflächig mit der Innenstützfläche 31 an der äußeren Oberfläche 25 des Innenrohrs 21 und mit der Außenstützfläche 32 an der inneren Oberfläche 26 des Außenrohrs 22 an. Bei den nachfolgend anhand der Figuren 4 bis 7 dargestellten Ausführungsformen ist jeweils zumindest entweder die Innenstützfläche 31 oder die Außenstützfläche 32 deutlich kleiner.

Die Figuren 4 bis 7 zeigen schematische Darstellungen von Querschnitten A-A von Varianten des Schafts 11 des oben anhand der Figur 1 dargestellten Endoskops 10. In jeder der Figuren 4 bis 7 sind jeweils links ein Querschnitt des gesamten Schafts 11 und rechts ein Querschnitt lediglich der Positionierungseinrichtung 30 dargestellt.

Bei den in den Figuren 4, 5 und 7 dargestellten Varianten sind das Innenrohr 21 und das Außenrohr 22 koaxial zueinander angeordnet, ähnlich wie bei der oben anhand der Figur 2 dargestellten Ausführungsform. Bei der in Figur 6 dargestellten Ausführungsform sind die Symmetrieachsen des Innenrohrs 21 und des Außenrohrs 22 parallel zueinander und voneinander beabstandet, ähnlich wie bei der oben anhand der Figur 3 dargestellten Ausführungsform.

Die in Figur 4 gezeigte Ausführungsform unterscheidet sich von der oben anhand der Figur 2 dargestellten Ausführungsform dadurch, dass die Positionierungseinrichtung 30 anstelle einer zusammenhängenden Außenstützfläche drei kleine Außenstützflächen 32 aufweist. Diese sind über Stege 34 mit einer großen, zusammenhängenden Innenstützfläche 31 verbunden. Die Stege 34 sind im Wesentlichen radial angeordnet und weisen einen gegenseitigen Abstand von ca. 120° auf. Bogenförmige Abschnitte der Stützeinrichtung 30 zwischen den Stegen 34 bilden aufgrund ihrer elastischen Eigenschaften Abschnitte einer Klemmeinrichtung 33.

Die Innenstützfläche 31 ist größer oder wesentlich größer als die drei Außenstützflächen 32 zusammen. Deshalb ist die Haftreibung zwischen der Innenstützfläche 31 und der äußeren Oberfläche 25 des Innenrohrs 21 größer oder wesentlich größer als die Haftreibung zwischen den Außenstützflächen 32 und der inneren Oberfläche 26 des Außenrohrs 22. Dies kann durch eine entsprechende Vorspannung der Positionierungseinrichtung 30 bzw. der Klemmeinrichtung 33 begünstigt werden. Dadurch eignet sich die in Figur 4 gezeigte Positionierungseinrichtung besonders für eine Klemmung am Innenrohr 21 vor dem Einführen des Innenrohrs 21 in das Außenrohr 22.

Die in Figur 5 gezeigte Ausführungsform unterscheidet sich von der oben anhand der Figur 2 dargestellten Ausführungsform dadurch, dass anstelle einer großen, zusammenhängenden Innenstützfläche drei kleine Innenstützflächen 31 vorgesehen sind. Eine einzige große, zusammenhängende Außenstützfläche 32 ist über drei Stege 34 mit jeweils einer der Innenstützflächen 31 verbunden. Jeder der Stege 34 ist im Wesentlichen radial angeordnet. Der Abstand zwischen zwei Stegen 34 beträgt jeweils ca. 120°. Bogenförmige Abschnitte zwischen den Stegen 34 bilden aufgrund ihrer elastischen Eigenschaften Abschnitte einer Klemmeinrichtung 33.

Die Außenstützfläche 32 ist größer oder wesentlich größer als die drei Innenstützflächen 31 zusammen. Deshalb ist die Haftreibung zwischen der Außenstützfläche 32 und der inneren Oberfläche 26 des Außenrohrs 22 größer oder wesentlich größer als die Haftreibung zwischen den Innenstützflächen 31 und der äußeren Oberfläche 25 des Innenrohrs 21. Dies kann durch eine entsprechende Vorspannung der Positionierungseinrichtung 30 bzw. der Klemmeinrichtung 33 begünstigt werden. Dadurch eignet sich die in Figur 5 gezeigte Positionierungseinrichtung besonders zur Klemmung im Außenrohr 22 vor dem Einführen des Innenrohrs 21 in das Außenrohr 22.

Die in Figur 6 gezeigte Ausführungsform unterscheidet sich von der oben anhand der Figur 3 dargestellten Ausführungsform dadurch, dass die Außenstützfläche 32 an zwei Stellen unterbrochen ist. An zwei Enden der Positionierungseinrichtung 30 ist jeweils ein Ende der Innenstützfläche 31 über einen Steg 34 mit einer kleinen Außenstützfläche 32 verbunden. Die beiden Stege 34 sind jeweils im Wesentlichen radial angeordnet. In einem mittleren Bereich weisen die Innenstützfläche 31 und eine große Außenstützfläche 32 aufgrund der exzentrischen Anordnung von Innenrohr 21 und Außenrohr 22 einen nur geringen gegenseitigen Abstand auf.

Ähnlich wie bei der oben anhand der Figur 4 dargestellten Ausführungsform ist die Innenstützfläche 31 deutlich größer als die Außenstützflächen 32 zusammen. Damit ist die Haftreibung zwischen der Innenstützfläche 31 und der äußeren Oberfläche 25 des Innenrohrs 21 größer als die Haftreibung zwischen den Außenstützflächen 32 und der inneren Oberfläche 26 des Außenrohrs 22. Deshalb eignet sich die in Figur 6 gezeigte Positionierungseinrichtung besonders zur Klemmung am Innenrohr 21 vor dem Einführen in das Außenrohr 22.

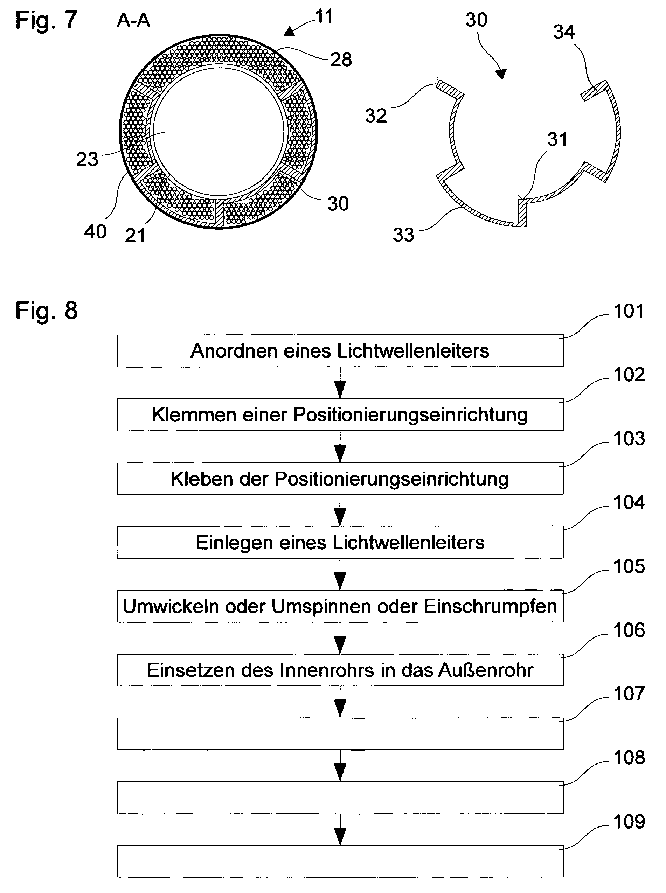

Figur 7 zeigt eine Ausführungsform, die sich von den oben anhand der Figuren 4 und 5 dargestellten Ausführungsformen dadurch unterscheidet, dass mehrere in umfänglicher Richtung voneinander beabstandete Innenstützflächen 31 und mehrere voneinander in umfänglicher Richtung beabstandete Außenstützflächen 32 alternierend bzw. abwechselnd angeordnet sind. Stege 34 verbinden jeweils im Wesentlichen in radialer Richtung eine Innenstützfläche 31 und eine Außenstützfläche 32. Gekrümmte Abschnitte der Positionierungseinrichtung 30 zwischen den Stegen 34 bilden aufgrund ihrer Elastizität Abschnitte einer Klemmeinrichtung 33.

Die Darstellung in Figur 7 unterscheidet sich von den Darstellungen der Figuren 2 bis 6 ferner dadurch, dass anstelle des Außenrohrs ein Mantel 40 dargestellt ist, der das Innenrohr 21 mit der Positionierungseinrichtung 30 und den Lichtwellenleitern 28 umschließt. Der Mantel 40 fixiert die Positionierungseinrichtung 30 und die Lichtwellenleiter 28 am Innenrohr 21 bevor das Innenrohr 21 mit der Positionierungseinrichtung 30 und den Lichtwellenleitern 28 in das in Figur 7 nicht dargestellte Außenrohr eingeführt wird. Der Mantel 40 ist beispielsweise ein Schrumpfschlauch, der auf das Innenrohr 21, die Positionierungseinrichtung 30 und die Lichtwellenleiter 28 aufgeschrumpft ist. Alternativ ist der Mantel beispielsweise durch Umwickeln oder Umspinnen des Innenrohrs 21, der Positionierungseinrichtung 30 und der Lichtwellenleiter 28 mit einem Faden, einem Draht, einem Band oder einer faden- oder draht- oder bandartigen Einrichtung hergestellt.

Es ist erkennbar, dass die oben anhand der Figuren 4 bis 7 dargestellten Positionierungseinrichtungen jeweils einen geringeren Anteil des Zwischenraums zwischen dem Innenrohr 21 und dem Außenrohr 22 einnehmen als die oben anhand der Figuren 2 und 3 dargestellten Positionierungseinrichtungen 30. Deshalb verbleibt mehr Raum für Lichtwellenleiter 28, elektrische Leitungen oder Kabel und andere Einrichtungen als bei den oben anhand der Figuren 2 und 3 dargestellten Ausführungsformen.

Positionierungseinrichtungen 30, wie sie oben anhand der Figuren 2 bis 7 dargestellt wurden, können in beliebiger Anzahl über die Länge des Schafts 11 eines Endoskops 10 verteilt angeordnet sein. Für viele Anwendungen ist die Anordnung einer Positionierungseinrichtung in der (bezogen auf die Längsrichtung des Schafts 11) Mitte des Schafts 11 vorteilhaft. Vor allem im Fall eines langen Schafts 11 können mehrere Positionierungseinrichtungen 30 über dessen Länge verteilt vorgesehen sein. Die Abstände zwischen benachbarten Positionierungseinrichtungen können gleich oder voneinander verschieden sein. Beispielsweise können in der Mitte des Schafts 11 kleinere Abstände zwischen den Positionierungseinrichtungen 30 vorgesehen sein als am distalen Ende 12 oder am proximalen Ende 13.

Eine Positionierungseinrichtung 30, wie sie oben anhand der Figuren 2 bis 7 dargestellt wurde, weist beispielsweise einen Kunststoff oder ein Metall auf und ist beispielsweise durch ein Gussverfahren oder mittels eines spanabhebenden Verfahrens (insbesondere Drehen oder Fräsen) hergestellt. Auch eine Herstellung aus einem entsprechenden Profil (beispielsweise Strangpressprofil), das in Abschnitte entsprechender Länge gesägt oder geschnitten wird, kann vorteilhaft sein.

Bei allen oben dargestellten Ausführungsformen hat der Schaft 11 des Endoskops 10 einen kreisförmigen Querschnitt. Die oben beschriebenen Positionierungseinrichtungen sind jedoch auch für ein Endoskop mit einem Schaft und mit Innen- und/oder Außenrohren geeignet, die nicht kreiszylindrisch sind.

Figur 8 zeigt ein schematisches Flussdiagramm eines Verfahrens zum Positionieren eines Innenrohrs in einem Außenrohr eines Endoskops. Obwohl dieses Verfahren auch auf Positionierungseinrichtungen und Endoskope anwendbar ist, die sich von den oben anhand der Figuren 1 bis 7 dargestellten unterscheiden, werden nachfolgend beispielhaft Bezugszeichen aus den Figuren 1 bis 7 verwendet, um das Verständnis zu erleichtern.

Bei einem ersten Schritt 101 wird ein Lichtwellenleiter 28 an einem Innenrohr 21 angeordnet. Bei einem zweiten Schritt 102 wird eine Positionierungseinrichtung 30 an das Innenrohr 21 geklemmt. Dabei werden der oder die beim ersten Schritt 101 am Innenrohr 21 angeordneten Lichtwellenleiter 28 am Innenrohr 21 zumindest lose fixiert.

Bei einem optionalen dritten Schritt 103 wird die Positionierungseinrichtung 30 durch Kleben, Löten, Schweißen oder auf andere Weise an dem Innenrohr 21 befestigt.

Alternativ zum ersten Schritt 101 oder zusätzlich zu diesem, werden bei einem vierten Schritt 104 nach dem dritten Schritt 103 Lichtwellenleiter 28 am Innenrohr 21 angeordnet. Dabei können die Lichtwellenleiter 28 zwischen dem Innenrohr 21 und der Positionierungseinrichtung 30 oder die Positionierungseinrichtung 30 zwischen dem Innenrohr 21 und den Lichtwellenleitern 28 angeordnet sein.

Bei einem optionalen fünften Schritt 105 wird ein Mantel um das Innenrohr 21, die Positionierungseinrichtung 30 und den oder die Lichtwellenleiter 28 gebildet. Der Mantel 40 wird beispielsweise durch Aufschrumpfen eines Schrumpfschlauchs oder durch Umwickeln oder Umspinnen mit einem oder mehreren Fäden, Drähten, Bändern oder faden- oder draht- oder bandartigen Einrichtungen gebildet.

Bei einem sechsten Schritt 106 wird das Innenrohr 21 in ein Außenrohr 22 eingesetzt.

Alternativ zum Klemmen der Positionierungseinrichtung 30 am Innenrohr 21 kann beim zweiten Schritt 102 die Positionierungseinrichtung 30 in das Außenrohr 22 eingesetzt und dort durch Klemmen und optional durch Kleben, Löten oder Schweißen fixiert werden bevor das Innenrohr 21 in das Außenrohr 22 eingesetzt wird. Vor oder beim Klemmen der Positionierungseinrichtung 30 im Außenrohr 22 können eine oder mehrere Lichtwellenleiter 28 zwischen der inneren Oberfläche 26 des Außenrohrs 22 und der Positionierungseinrichtung 30 angeordnet und zumindest lose fixiert werden.

### Bezugszeichen

- 10: Endoskop
- 11: Schaft des Endoskops 10
- 12: distales Ende des Endoskops 10
- 13: proximales Ende des Endoskops 10
- 14: Okular des Endoskops 10
- 15: Kupplung für Lichtquelle
- 21: Innenrohr des Schafts 11
- 22: Außenrohr des Schafts 11
- 23: Lumen des Innenrohrs 21
- 24: Zwischenraum zwischen Innenrohr 21 und Außenrohr 22
- 25: äußere Oberfläche des Innenrohrs 21
- 26: innere Oberfläche des Außenrohrs 22
- 28: Lichtwellenleiter
- 30: Positionierungseinrichtung
- 31: Innenstützfläche der Positionierungseinrichtung 30
- 32: Außenstützfläche der Positionierungseinrichtung 30
- 33: Klemmeinrichtung der Positionierungseinrichtung 30
- 34: Steg
- 40: Mantel

- 101: erster Schritt
- 102: zweiter Schritt
- 103: dritter Schritt
- 104: vierter Schritt
- 105: fünfter Schritt
- 106: sechster Schritt

## Patentansprüche

1. **Verfahren** zum Positionieren eines Innenrohrs (21) in einem Außenrohr (22) eines Endoskops (10), mit folgenden Schritten:
**Klemmen (102)** einer Positionierungseinrichtung (30) am Innenrohr (21) oder am Außenrohr (22), wobei die Positionierungseinrichtung (30) eine Innenstützfläche (31) zur Abstützung am Innenrohr (21) und eine Außenstützfläche (32) zur Abstützung am Außenrohr (22) aufweist;
**Anordnen (106)** des Innenrohrs (21) im Außenrohr (22) nach dem Klemmen (102) der Positionierungseinrichtung (30),
**wobei** die Positionierungseinrichtung (30) ausgebildet ist, um das Innenrohr (21) zu mehr als der Hälfte zu **umgreifen,**
**wobei** das Innenrohr (21) und das Außenrohr (22) koaxial bzw. konzentrisch zueinander angeordnet sind, und die Positionierungseinrichtung (30) damit insgesamt die Gestalt eines unvollständigen Kreisrings mit rechteckigem Querschnitt hat,
**wobei** die Positionierungseinrichtung (30) einen **Steg (34) aufweist,** der in radialer Richtung eine der zumindest einen Innenstützfläche (31) und eine der zumindest einen Außenstützfläche (32) verbindet, ferner mit folgendem Schritt:
Anordnen (101; 104) eines **Lichtwellenleiters (28)** an der Außenwand (25) des Innenrohrs (21) oder an der Innenwand des Außenrohrs (22) vor dem Klemmen (102) der Positionierungseinrichtung (30).

2. Verfahren nach dem vorangehenden Verfahrensanspruch, ferner mit folgendem Schritt:
Aufschrumpfen (105) eines **Schrumpfschlauchs (40)** auf das Innenrohr (21) und die Positionierungseinrichtung (30) nach dem Klemmen (102) der Positionierungseinrichtung (30) am Innenrohr (21).

3. Verfahren nach einem der vorangehenden Verfahrensansprüche, ferner mit folgendem Schritt:
**Umwickeln** (105) oder Umspinnen des Innenrohrs (21) und der Positionierungseinrichtung (30) nach dem Klemmen (102) der Positionierungseinrichtung (30) am Innenrohr (21).

4. Verfahren nach einem der vorangehenden Verfahrensansprüche, ferner mit folgendem Schritt:
**Stoffschlüssiges Verbinden (103)** der Positionierungseinrichtung (30) mit dem Innenrohr (21) oder mit dem Außenrohr (22).

5. **Endoskop (10)** mit einer Positionierungseinrichtung (30) zur Positionierung eines Innenrohrs (21) in einem Außenrohr (22) des Endoskops (10), wobei:
die Positionierungseinrichtung (30) eine Innenstützfläche (31) zur Abstützung am Innenrohr (21) und eine Außenstützfläche (32) zur Abstützung am Außenrohr (22) aufweist und
die Positionierungseinrichtung (30) am Innenrohr (21) oder am Außenrohr (22) geklemmt ist,
**wobei** die Positionierungseinrichtung (30) das Innenrohr (21) zu mehr als der Hälfte **umgreift,**
**wobei** das Innenrohr (21) und das Außenrohr (22) koaxial bzw. konzentrisch zueinander angeordnet sind und die Positionierungseinrichtung (30) damit insgesamt die Gestalt eines unvollständigen Kreisrings mit rechteckigem Querschnitt hat,
wobei die Positionierungseinrichtung (30) einen **Steg (34) aufweist,** der in radialer Richtung eine der zumindest einen Innenstützfläche (31) und eine der zumindest einen Außenstützfläche (32) verbindet, und
ein **Lichtwellenleiter (28)** an der Außenwand (25) des Innenrohrs (21) oder an der Innenwand des Außenrohrs (22) angeordnet ist.

6. **Endoskop (10)** nach dem vorangehenden Anspruch, wobei die Positionierungseinrichtung (30) **mehrere** von einander beabstandete **Innenstützflächen (31) aufweist.**

7. **Endoskop (10)** nach einem der Ansprüche 5 oder 6, wobei
die Positionierungseinrichtung (30) **mehrere** von einander beabstandete **Außenstützflächen (32) aufweist.**

8. **Endoskop (10)** nach einem der Ansprüche 5 bis 7, wobei
zumindest eine **Innenstützfläche (31)** und zumindest eine **Außenstützfläche (32)** in umfänglicher Richtung **abwechselnd** angeordnet sind.

9. Endoskop (10) nach einem der Ansprüche 5 bis 8, ferner mit:
einem **Schrumpfschlauch (40),** der auf das Innenrohr (21) und die Positionierungseinrichtung (30) aufgeschrumpft ist.

10. Endoskop (10) nach Anspruch 5 bis 8, bei dem das Innenrohr (21) und die Positionierungseinrichtung (30) **umwickelt** oder **umsponnen** sind.

## Claims

1. Method for positioning an inner tube (21) in an outer tube (22) of an endoscope (10), with the following steps:
clamping (102) a positioning means (30) on the inner tube (21) or on the outer tube (22), wherein the positioning means (30) has an inner support surface (31) for supporting on the inner tube (21) and an outer support surface (32) for supporting on the outer tube (22);
arranging (106) the inner tube (21) in the outer tube (22) after the clamping (102) of the positioning means (30),
wherein the positioning means (30) is designed to engage more than half way round the inner tube (21),
wherein the inner tube (21) and the outer tube (22) are arranged coaxially or concentrically with respect to each other, and the positioning means (30) thus overall has the shape of an incomplete annulus with a rectangular cross section,
wherein the positioning means (30) has a web (34) which, in a radial direction, connects one of the at least one inner support surface (31) and one of the at least one outer support surface (32),
moreover with the following step:
arranging (101; 104) an optical waveguide (28) on the outer wall (25) of the inner tube (21) or on the inner wall of the outer tube (22) before the clamping (102) of the positioning means (30).

2. Method according to the preceding method claim, moreover with the following step:
shrink-fitting (105) a shrink-fit hose (40) onto the inner tube (21) and the positioning means (30) after the clamping (102) of the positioning means (30) on the inner tube (21).

3. Method according to either of the preceding method claims, moreover with the following step:
winding (105) or braiding material around the inner tube (21) and the positioning means (30) after the clamping (102) of the positioning means (30) on the inner tube (21).

4. Method according to one of the preceding method claims, moreover with the following step:
cohesively connecting (103) the positioning means (30) to the inner tube (21) or to the outer tube (22).

5. Endoscope (10) with a positioning means (30) for positioning an inner tube (21) in an outer tube (22) of the endoscope (10), wherein:
the positioning means (30) has an inner support surface (31) for supporting on the inner tube (21) and an outer support surface (32) for supporting on the outer tube (22), and
the positioning means (30) is clamped on the inner tube (21) or on the outer tube (22),
wherein the positioning means (30) engages more than half way round the inner tube (21),
wherein the inner tube (21) and the outer tube (22) are arranged coaxially or concentrically with respect to each other, and the positioning means (30) thus overall has the shape of an incomplete annulus with a rectangular cross section,
wherein the positioning means (30) has a web (34) which, in a radial direction, connects one of the at least one inner support surface (31) and one of the at least one outer support surface (32), and an optical waveguide (28) is arranged on the outer wall (25) of the inner tube (21) or on the inner wall of the outer tube (22).

6. Endoscope (10) according to the preceding claim, wherein the positioning means (30) has a plurality of inner support surfaces (31) spaced apart from each other.

7. Endoscope (10) according to either of Claims 5 and 6, wherein the positioning means (30) has a plurality of outer support surfaces (32) spaced apart from each other.

8. Endoscope (10) according to one of Claims 5 to 7, wherein at least one inner support surface (31) and at least one outer support surface (32) are arranged in alternating fashion in the circumferential direction.

9. Endoscope (10) according to one of Claims 5 to 8, moreover with:
a shrink-fit hose (40) which is shrink-fitted onto the inner tube (21) and the positioning means (30).

10. Endoscope (10) according to Claims 5 to 8, in which material is would or braided around the inner tube (21) and the positioning means (30).

## Revendications

1. Procédé de positionnement d'un tube intérieur (21) dans un tube extérieur (22) d'un endoscope (10), avec les étapes suivantes :
serrage (102) d'un dispositif de positionnement (30) au niveau du tube intérieur (21) ou au niveau du tube extérieur (22), le dispositif de positionnement (30) comportant une surface de maintien intérieure (31) pour le maintien au niveau du tube intérieur (21) et une surface de maintien extérieure (32) pour le maintien au niveau du tube extérieur (22) ;
agencement (106) du tube intérieur (21) dans le tube extérieur (22) après le serrage (102) du dispositif de positionnement (30) ;
le dispositif de positionnement (30) étant réalisé pour enserrer de plus de la moitié le tube intérieur (21) ;
le tube intérieur (21) et le tube extérieur (22) étant disposés dans le plan coaxial et/ou concentriquement l'un par rapport à l'autre et le dispositif de positionnement (30) ayant ainsi dans l'ensemble la forme d'une couronne circulaire non entière de section transversale rectangulaire ;
le dispositif de positionnement (30) comportant un étai (34) reliant dans la direction radiale au moins une surface de maintien intérieure (31) et au moins une surface de maintien extérieure (32) ;
en outre avec l'étape suivante :
agencement (101 ; 104) d'un conducteur d'ondes lumineuses (28) au niveau de la paroi extérieure (25) du tube intérieur (21) ou au niveau de la paroi intérieure du tube extérieur (22) avant le serrage (102) du dispositif de positionnement (30).

2. Procédé selon la revendication de procédé précédente, avec en outre l'étape suivante :
rétractation (105) d'un tuyau flexible rétractable (40) sur le tube intérieur (21) et le dispositif de positionnement (30) après le serrage (102) du dispositif de positionnement (30) au niveau du tube intérieur (21).

3. Procédé selon l'une quelconque des revendications de procédé précédentes, avec en outre l'étape suivante :
enroulement (105) ou brochage du tube intérieur (21) et du dispositif de positionnement (30) après le serrage (102) du dispositif de positionnement (30) au niveau du tube intérieur (21).

4. Procédé selon l'une quelconque des revendications de procédé précédentes, avec en outre l'étape suivante :
liaison par complémentarité de matières (103) du dispositif de positionnement (30) avec le tube intérieur (21) ou avec le tube extérieur (22).

5. Endoscope (10) avec un dispositif de positionnement (30) pour le positionnement d'un tube intérieur (21) dans un tube extérieur (22) de l'endoscope (10) :
le dispositif de positionnement (30) comportant une surface de maintien intérieure (31) pour le maintien au niveau du tube intérieur (21) et une surface de maintien extérieure (32) pour le maintien au niveau du tube extérieur (22) ; et
le dispositif de positionnement (30) étant serré au niveau du tube intérieur (21) ou au niveau du tube extérieur (22) ;
le dispositif de positionnement (30) enserrant le tube intérieur (21) de plus de la moitié ;
le dispositif de positionnement (30) étant réalisé pour enserrer de plus de la moitié le tube intérieur (21) ;
le tube intérieur (21) et le tube extérieur (22) étant disposés dans le plan coaxial et/ou concentriquement l'un par rapport à l'autre et le dispositif de positionnement (30) ayant ainsi dans l'ensemble la forme d'une couronne circulaire non entière de section transversale rectangulaire ;
le dispositif de positionnement (30) comportant un étai (34) reliant dans la direction radiale au moins une surface de maintien intérieure (31) et au moins une surface de maintien extérieure (32) ; et
un conducteur d'ondes lumineuses (28) étant disposé au niveau de la paroi extérieure (25) du tube intérieur (21) ou au niveau de la paroi intérieure du tube extérieur (22).

6. Endoscope (10) selon la revendication précédente, le dispositif de positionnement (30) comportant plusieurs surfaces de maintien intérieures (31) espacées les unes par rapport aux autres.

7. Endoscope (10) selon l'une quelconque des revendications 5 ou 6, le dispositif de positionnement (30) comportant plusieurs surfaces de maintien extérieures (32) espacées les unes par rapport aux autres.

8. Endoscope (10) selon l'une quelconque des revendications 5 à 7, au moins une surface de maintien intérieure (31) et au moins une surface de maintien extérieure (32) étant disposées alternativement dans la direction périphérique.

9. Endoscope (10) selon l'une quelconque des revendications 5 à 8, en outre avec :
un tuyau flexible rétractable (40) se rétractant sur le tube intérieur (21) et le dispositif de positionnement (30) .

10. Endoscope (10) selon la revendication 5 à 8, dans lequel le tube intérieur (21) et le dispositif de positionnement (30) sont enroulés ou serrés autour.
